# FASCICULE DE BREVET EUROPEEN

(11) **EP 0 820 273 B1**
(45) Date de publication et mention de la délivrance du brevet: **01.10.2003**
(21) Numéro de dépôt: 95923372.7
(22) Date de dépôt: 12.06.1995
(51) Int. Cl.: A61K 7/50, A61K 7/48, A61K 7/06, A61K 31/70

(54) **UTILISATION DES SOPHOROLIPIDES DANS DES COMPOSITIONS COSMETIQUES ET DERMATOLOGIQUES**
VERWENDUNG VON SOPHOROSELIPIDEN IN KOSMETISCHEN UND DERMATOLOGISCHEN ZUSAMMENSETZUNGEN
USE OF SOPHOROLIPIDS IN COSMETIC AND DERMATOLOGICAL COMPOSITIONS

(30) Priorité: 13.06.1994 FR 9407287
(43) Date de publication de la demande: 28.01.1998
(73) Titulaire: SOPHOR S.A., 92500 Reuil-Malmaison (FR)
(72) Inventeur: HILLION, Gérard, F-95220 Herblay (FR); MARCHAL, Rémy, F-78400 Chatou (FR); STOLTZ, Corinne, F-77190 Dammarie-les-Lys (FR); BORZEIX, Frédérique, F-92500 Rueil-Malmaison (FR)
(86) Numéro de dépôt international: FR9500768
(87) Numéro de publication internationale: WO95034282

(56) Documents cités:
- EP-A- 0 209 783
- DATABASE WPI Week 8008, Derwent Publications Ltd., London, GB; AN 80-13763C 'Purification of sophorolipid derivatives useful in cosmetics.' & JP,A,55 004 344 (KAO SOAP KK) 12 Janvier 1980

## Description

La présente invention concerne une nouvelle utilisation des sophorolipides.

La présente invention concerne également des compositions cosmétologiques, ou dermatologiques.

Plus particulièrement, la présente invention concerne de telles compositions à propriétés anti-radicalaires et anti-élastasiques. Ces propriétés sont particulièrement utiles dans des compositions destinées aux soins de la peau et des cheveux.

Les sophorolipides sont des glycolipides ; ils sont produits par fermentation de levures de type *Candida ou Torulopsis*, telles que *Toruiopsis magnoliae, Candida bambicola, Candida apicola* ou *Candida bogoriensis*.

Le glucide constitutif des sophorolipides extracellulaires est le sophorose (2' - O - β - D - glucopyranosyl - β - D - glucopyranose). Ce sucre peut être acétylé en position 6' et 6" et il est relié à un hydroxy-acide gras en position ω ou ω - 1.

La traction lipidique des sophorolipides, composée de plusieurs hydroxy-acides gras, qui différent par la longueur de leur chaine, par le nombre et la position des insaturations, ainsi que par la position de l'hydroxylation, dépend de la composition lipidique de la source secondaire de carbone utilisé dans la fermentation (huile végétale plus ou moins riche en lipides saturés ou insaturés).

Ces glycolipides sont synthétisés sous forme d'un mélange de plusieurs classes de sophorolipides, qui différent par leur degre de saturation, ou d'insaturation, par l'absence ou la présence de plusieurs types de lactonisation et par différents degrés d'acétylation.

Les différentes classes de sophorolipides sont les suivantes:
1. Lactones, acétylées ou non, en position 6' et 6" du sophorose:
   - sophorolipide 1',4" - lactone 6' - 6" - diacétate (notation: S1 - 4" L6'a 6"a),
   - sophorolipide 1',4" - lactone 6" - mono-acétate (notations : S1 - 4" L6"a),
   - sophorolipide 1,4" - lactone (notation: S1 - 4" L).
2. Acides, acétylés ou non, en position 6' du sophorose:
   - sophorolipide acide 6' - mono-acétate (notation: S1 - A6'a),
   - sophorolipide acide (notation: S1 - A).

Deux formules chimiques représentatives des formes lactone et acide des sophorolipides sont indiquées ci-dessous: 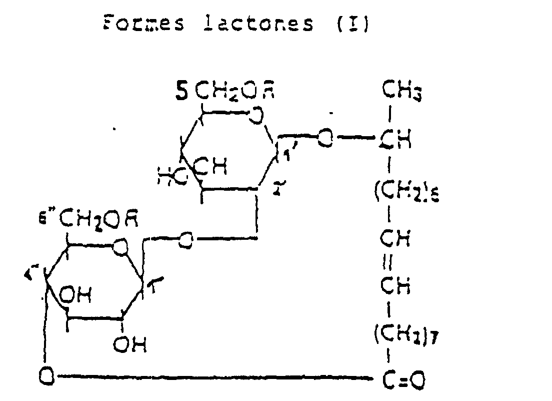 et : 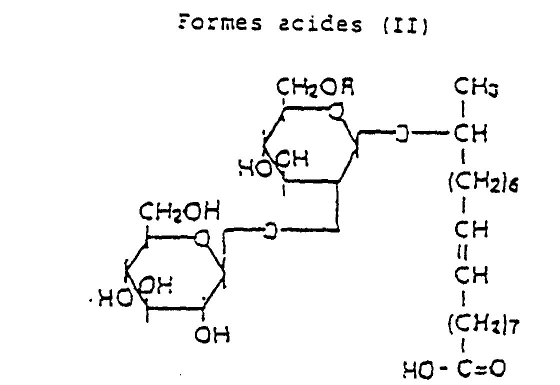 Formules dans lesquelles R = H ou CH3-CO

Ces formules chimiques illustrent le cas de l'hydroxy-acide gras constitutif le plus abondant, l'acide 17 - hydroxyoctadécénoïque (17 - hydroxyoléique).

Les contributions respectives de chacun des hydroxy-acides constitutifs déterminés en chromatographie phase gazeuse, aux différentes classes de sophorolipides, ainsi que la répartition de ces classes, sont indiquées ci-après.

Les hydroxy-acides gras constitutifs des sophorolipides sont les suivants, par exemple :

| Types | Hydroxy-acides gras | % |
|---|---|---|
| C 16:0 | 15-OH Hexadécanoïque | 1,5 |
| C 16:0 | 16-OH Hexadécanoïque | 2,0 |
| C 18:0 | 17-OH Octadécanoïque | 3,5 |
| C 18:1 | 17-OH Octadécénoïque | 60,0 |
| C 18:1 | 18-OH Octadécadiénoïque | 12,0 |
| C 18:2 | 17-OH Octadécadiénoïque | 7,0 |
| C 18:2 | 18-OH Occadécadiénoïque | 14,0 |

Les principales voies de production des glycolipides par biosynthèse se font à partir de triglycérides ou de carbohydrates.

La production des glycolipides par le micro-organisme ne nécessite pas d'apport supplémentaire d'azote et de sel; elle s'effectue tant que la cellule trouve une source de carbone et d'oxygène suffisante.

La biosynthèse des glycolipides comporte la synthèse de la partie lipophile (acide gras), ainsi que de la partie hydrophile (sophorose).

La biosynthèse des glycolipides est caractérisée par les facteurs suivants:
- la synthèse de la partie lipidique dépend de la source de carbone hydrophobe utilisée (huile) comme substrat;
- la synthèse de la partie glucidique dépend également de la source de carbone utilisée, mais est synthétisée de novo.

Les levures du genre *Torulopsis*, telles que *T. magnoliae* ou *T*. *bombicola* (classées à présent dans le genre *Candida*), excrètent des sophorolipides, si elles sont cultivées en présence de 10% de glucose, 1,25% d'extraits de levure et 0,2% d'urée, par exemple.

Des études ont montré que le taux da production de sopholipides peut étre augmenté par addition, dans le milieu de culture, d'acides gras estérifiés à longue chaine, ou de n-alcanes, ou de triglycérides.

Des procédés de production de sophorolipides acétylés sous leur forme acide par culture d'une souche de *Candida bombicola* ou *Candida apicola*, utilisent notamment un substrat consistant en une huile végétale.

Les agents tensio-actifs sont utilisés pour les soins du corps, les soins dentaires, dans les agents lavants et ceci aussi bien dans le domaine pharmaceutique que cosmétique. Au vu de leur utilisation grandissante, les considérations écologiques actuelles ont amené à étudier les propriétés biologiques et toxiques de ces substances. Ainsi on sait que les surfactants synthétiques (donc non biologiques), modifient la perméabilité des membranes biologiques. Les surfactants cationiques et anioniques agissent sur la libération de molécules intracellulaires ou en inhibent la respiration. Les surfactants synthétiques, en fonction de leur concentration, peuvent également avoir une action toxique.

Un objet de la présente invention est de proposer des agents tensio-actifs biologiques non toxiques dans les compositions cosmétiques ou pharmaceutiques.

Les sophorolipides ont été utilisés comme détergent industriel biodégradable, notamment dans des procédés de dépollution tel que décrit dans les demandes de brevet en France n'^{s} 92/16033 et 93/14999 et la demande de brevet Européen EP 499 434.
- La demande de brevet EP-A-209 783 (Wella) décrit la forme lactone des sophorolipides et son utilisation en tant qu'agent antipelliculaire et bactériostatique.
- La demande de brevet JP-A-55 004 344 (Kao) mentionne un ester d'hydroxyalkyl éther glycolipide utilisé en cosmétologie.

Aucune de ces demandes ne décrit ni ne suggère une composition à propriété anti-radicalaire, anti-élastasique et/ou anti-inflammatoire.

De plus, il a été proposé des compositions cosmétiques et pharmaceutiques à bases de glycolipides tels que des glycosylcéramides ou digalactodiglycérides pour apporter le bénéfice des lipides à la peau, notamment à des fins d'hydratation de la peau.

On a découvert selon la présente invention que les sophorolipides sont des glycolipides dotés de propriétés physico-chimiques avantageuses, notamment de propriétés émulsifiantes multiples qui permettent de préparer des formulations très diverses à base de sophorolipides utilisables en cosmétique ou en applications topiques en général.

D'autre part, on a découvert que ces sophorolipides, outre qu'ils apportent à la peau tout le bénéfice des lipides, sont dotés de propriétés anti-radicalaires et anti-élastasique, propriétés qui sont précisément recherchées dans les compositions à usage cosmétique, hygiénique ou pharmaco-dermatologique, en particulier pour la protection et le soin des cheveux ou de la peau.

La présente invention a donc pour objet l'utilisation de sophorolipides pour la préparation d'une composition cosmétologique ou dermatologique et la composition obtenue.

La présente invention propose donc, pour la première fois, des glycolipides tensioactifs d'origine biologique à propriétés émulsifiantes multiples et ayant des propriétés anti-radicalaires, anti-élastasiques et/ou anti-inflammatoires.

Selon la présence invention, les sophorolipides permettent de par leurs propriétés émulsifiantes particulières de préparer des compositions cosmétologiques sous forme d'émulsions à phase continue aqueuse (émulsion huile dans l'eau) ou à phase continue huileuse (émulsion eau dans l'huile) qui apportent à la peau ou aux cheveux des lipides sans recourir nécessairement à l'usage de tensio-actifs et émulsifiants synthétiques. Ces compositions sont particulièrement utiles comme vecteurs de principe actif de médicaments.

Selon la présente invention, les sophorolipides permettent en outre d'obtenir des émulsions à phase continue huileuse stable dont la phase oléophile peut être entièrement d'origine végétale quand le substrat utilisé dans la production des sophorolipides par fermentation est une huile végétale et quand la phase oléphile comporte une huile végétale telle de l'huile d'olive, huile d'amande douce, huile de noix ou de l'huile de coco hydrogénée ou autres. On sait en effet qu'il est particulièrement recherché d'utiliser des produits d'origine végétale plutôt qu'animale de façon à éviter les problèmes toxicologiques.

Enfin, lorsqu'ils sont utilisés sous forme acide ou de sel métallique monovalent, tel qu'un sel de sodium ou de potassium ou divalent dudit acide, les sophorolipides constituent un tensio-actif anionique qui présente des valeurs de HLB variables selon le pH, grâce à quoi il est possible en particulier d'obtenir pour des valeurs de HLB élevées, notamment d'environ 8 à 12, des émulsions aqueuses et même des microémulsions aqueuses stables et d'une grande limpidité (HLS d'environ 13 à 15), ce qui est une propriété recherchée pour la préparation de certaines compositions cosmétologiques et hygiéniques.

En particulier, les microemulsions aqueuses (microémulsion d'huile dans l'eau) sont intéressantes pour obtenir des lotions parfumantes et eaux de toilette sans utiliser de solution hydroalcoolique, c'est-à-dire sans utiliser d'alcool.

Dans un mode de réalisation avantageux de la présente invention, les compositions comportent comme seul agent émulsifiant un sophorolipide. Toutefois, un effet synergique peut être aussi obtenu por un mélange d'un tensio-actif convéntionnel tel que le dioctylsulfosuccinate de sodium (DOS), le nonylphénoléthoxylé, le monolaurate de sorbitan éthoxylé (Tween 20) ou le cétyl triméthyl anmonium, et les sophorolipides sur l'abaissement de la tension interfaciale entre l'eau et les hydrocarbures.

Toutefois, dans les compositions selon l'invention, les sophorolipides sont de préférence purifiés afin d'obtenir uniquement les sophorolipides sous leur forme acide et/ou leurs sels, laquelle est plus stable et plus soluble que les formes lactones. La stabilité est importante au niveau olfactif. Les formes lactones sont particulièrement peu solubles en solution aqueuse alors que les formes acides et/ou leurs sels sont solubles en solution aqueuse.
De préférence, les sophorolipides utilisés selon la présente invention sont obtenus par fermentation de *C. bogoriensis* ou *C, bombicola*. On cite en particulier les sophorolipides qui répondent aux formules [1] (forme acide) et [2] (forme lactone) présentées ci-après, dans lesquelles R¹ représente l'hydrogène ou un groupe acétyle et R² l'hydrogène ou un groupe alkyle comportant 1 à 9 atomes de carbone, lorsque R³ est un reste hydrocarboné saturé à 7 à 16 atomes de carbone, ou bien R² représente l'hydrogène ou un groupe méthyle, lorsque R³ est un reste hydrocarboné insaturé à 13 à 17 atomes de carbone ; en 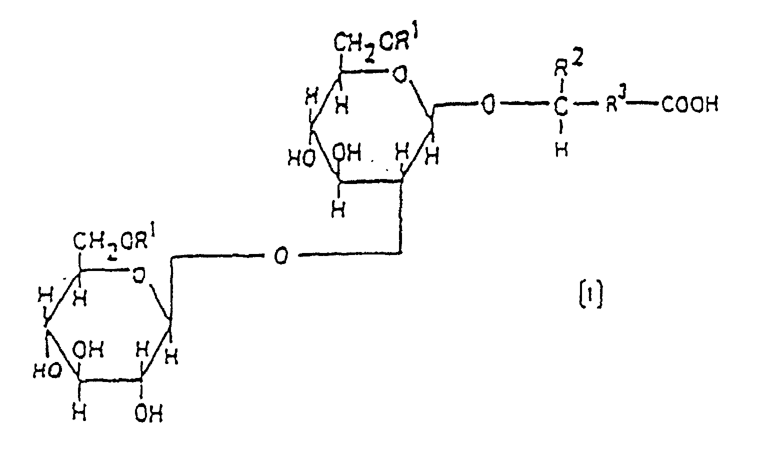 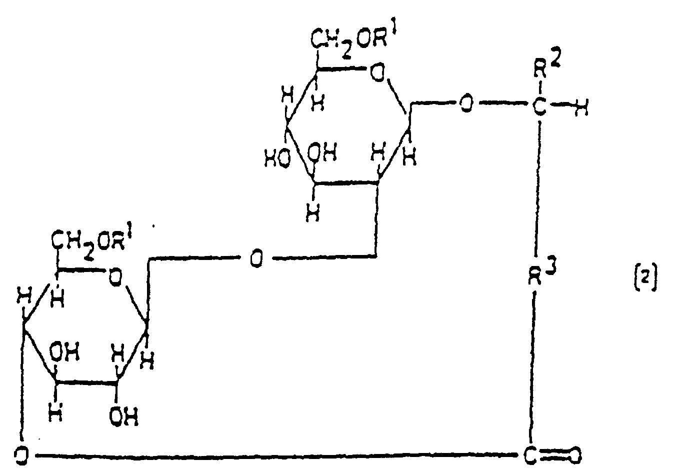 particulier, -R²CH- R³ - est un reste divalent d'un hydroxy-acide gras en C₁₆ ou C₁₈ saturé ou insaturé avec une ou deux double(s) liaison(s) tels que mentionnés précédemment.

Les compositions selon l'invention comportent de 0,01 à 30% (p/p) de sophorolipides (matière sèche) de la composition totale et plus particulièrement de 0,05 à 5% de sophorolipides (matière sèche).

Les compositions selon l'invention peuvent comporter des acides gras saturés ou non en C₁₀-C₃₄, de préférence C₁₄-C₂₂, tels que les acides oléiques, linoléiques, linoléniques, palmitique et palmitoléique de 0,01 à 20% en poids par rapport aux sophorolipides (matière sèche), plus particulièrement de 1 à 10%, une majeure partie pouvant être rajoutée ultérieurement en fonction des usages envisagés.

Dans des modes de réalisations particuliers, les compositions cosmétologiques selon l'invention consisteront en:
- des laits, notamment sous forme de lotions oléoalcooliques, émulsions aqueuses ou microémulsions aqueuses,
- des lotions, telles que lotions parfumantes, ou des eaux de toilette, notamment sous forme de lotions hydroalcooliques, ou microémulsions aqueuses stables,
- des shampooings, des gels de douche, des savons liquides sous forme de microémulsions aqueuses,
- des gels démaquillants, des crèmes hydratantes,
   notamment sous forme d'émulsions eau/huile ou huile/eau.
- des mélanges gras du type solide tel qu'un bâton de rouge à lèvres, ou une pommade.

Ces propriétés sont en particulier utiles pour les soins et l'hygiene capillaires et de la peau.

Les compositions cosmétologiques selon l'invention sont utiles pour les propriétés anti-radicalaires et ou anti-élastasiques des sophorolipides. Utilisées en dermatologie, elles présentent des propriétés anti-radicalaires, anti-élastasiques et/ou anti-inflammatoires.

Dans les compositions pharmaco-dermatologiques, on cite toutes les formulations de compositions administrables par voie topique, notamment les pommades, crèmes, laits, lotions, gels et les savons liquides.

D'autres avantages et caractéristiques de la présente apparaitront à la lumière des exemples qui vont suivre.

Les tableaux 10 et 11 représentent différentes formulations possibles de compositions selon l'invention.

### Exemple 1

On donne ici, à titre illustratif, les conditions de synthèse dans des souches de Candida bombicola.

Les conditions optimales de production de sophorolipides sont les suivantes :
- On utilise un milieu de culture contenant initialement 10% de glucose, 0,5% de liqueur de macération de maïs séchée et 0,4% de sulfate d'ammonium. Le pH du milieu de culture est initialement de 6,0 et la température d'incubation est fixée entre 22 et 30°C.
On additionne en continu une seconde source de carbone comme des alcanes, des alcools à longue chaîne carbonée, des acides gras ou leurs esters éthyliques ou méthyliques.
- En fin de fermentation, le rapport de consommation glucose/seconde source de carbone est en général d'environ 2:1.
- Avec *C. bombicola* CBS 6009, 300 g/l de sophorolipides peuvent être produits en utilisant une alimentation continue d'ester méthylique (ou éthylique) de colza et des ajouts quotidiens de glucose comme décrit dans le brevet français FR 2 692 593.
Les spécifications des sophorolipides forme brute et acide sont données ci-après :

### 1 - Sophorolipides forme brute

- aspect : liquide, visqueux
- odeur : peu marquée
- matière sèche ≤ 60 % ± 0,5 %
- couleur : ambrée
- acide gras libre : ≤ 4 % (2,6 % acide oléique)
- sucre libre ≤ 1 %
- sels minéraux ≤ 2 %
- pH : 7 ± 0,5
- conservateurs : phénoxyéthanol, parahydroxybenzoate de méthyl sodé
- densité à 20°C 1,097
   densité à 40°C 1,085
- viscosité cinématique : 562,61 mm2/s : à 20°C
   : 160,49mm2/s : à 40°C
La répartition des hydroxyacides gras constitutifs des sophorolipides forme brute est la suivante (tableau 1)

| Acides gras | % |
|---|---|
| 15-OH C16:0 | 1,7 |
| 16-OH C16:0 | 1,2 |
| 17-OH C18:2 | 6,7 |
| 17-OH C18:1 | 63,4 |
| 17-OH C18:0 | 3,2 |
| 17-OH C18:2 | 13,5 |
| 18-OH C18:1 | 10,3 |

### 2 - Les sophorolipides forme acide

Les sophorolipides forme acide sont obtenus après hydrolyse alcaline des sophorolipides forme brute.
- aspect : liquide peu visqueux
- odeur : légèrement acétylée
- teneur en eau : ≥ 60 % ± 5 %
- acide gras libre : ≤ 5 % (2,4 % acide oléique)
- hydroxyacide gras libre : ≤ 0,5 %
- sucre libre ≤ 1 %
- minéraux ≤ 2 %
- conservateurs : phénoxyéthanol, parahydroxybenzoate de méthyl sodé (POBM, Na)
- pH : 6 ± 0,5%
- densité à 20°C 1,092
   densité à 40°C 1,082
- viscosité cinématique : 96 mm²/s : à 20°C
   : 42 mm²/s : à 40°C

La répartition des hydroxyacides gras des sophorolipides forme acide est la suivante (tableau 2).

| Hydroxy-acides | quantité relative (%) |
|---|---|
| 15-OH C16:0 | 0,5 |
| 16-OH C16:0 | 1,0 |
| 17-OH C18:0 | 4,0 |
| 17-OH C18:1 | 64,0 |
| 18-OH C18:1 | 10,0 |
| 17-OH C18:2 | 7,0 |
| 18-OH C18:2 | 13,5 |

### Exemple 2

### 1 - Tolérance oculaire

Les produits sophorolipides forme brute à 6 % (madère sèche) et sophorolipides forme acide à 4 % (matière sèche) ont été instillés en une seule fois, à raison de 0.1ml dans l'oeil de trois lapins, selon un protocole établi à partir de la méthode officielle, définie par l'Arrêté du 9 Juin 1992 (Journal Officiel de la République Française du 10 Juillet 1992).

Les scores relevés ont permis d'obtenir un indice d'irritation maximum de 15.0 et 7.3 une heure après l'instillation, respectivement pour les formes brute et acide des sophorolipides.

Les résultats de ces essais permettent de conclure que les produits sophorolipides forme brute et sophorolipides forme acide sont faiblement irritants.

### 2 - Tolérance cutanée

Les sophorolipides forme brute à 6 % (matière sèche) et forme acide à 4 % (matière sèche), dilués dans l'eau distillée, ont été appliqués à la dose de 0.5 ml sur une zone de peau saine et sur une zone de peau scarifiée de trois lapins selon un protocole établi à partir de la méthode officielle définie par l'Arrêté du 1^{er} Février 1982 (Journal Officiel de la République Française du 21 Février 1982).

Les scores relevés ont permis d'obtenir un indice de 0.3 et 0.1 respectivement pour les sophorolipides forme brute et forme acide.

les résultats de ces essais permettent de conclure que les produits sophorolipides forme brute et sophorolipides forme acide sont non irritants.

### 3 - Innocuité par voie orale

L'objectif de l'étude réalisée selon le présent protocole, est d'évaluer la toxicité après administration orale unique d'une dose maximale de sophorolipides.

Les produits référencés sophorolipides forme brute et sophorolipides forme acide, ont été administrés à deux lots de rats mâles et femelles, à la dose de 5 ml/Kg.

La méthodolgie utilisée pour cette étude est celle dérivée du protocole inscrit dans la Directive O. C. D. E. 84/449 L251 du 25 Avril 1984.

Pour chaque produit testé séparément, la mortalité dans les deux lots d'animaux a été nulle.

Dans ces conditions, les produits sophorolipides forme brute et sophorolipides forme acide ont une innocuité par voie orale supérieure ou égale à 5 ml/Kg de poids.

### 4 - Détermination du pouvoir sensibilisant selon Magnusson et Kligman

L'objectif de l'étude réalisée selon le présent protocole, est d'évaluer le potentiel sensibilisant des sophorolipides forme brute et des sophorolipides forme acide sur le cobaye albinos. Deux semaines après une série de contacts répétés avec les produits à l'étude (phase d'induction) les cobayes sont soumis à une application déclenchante avec les mêmes produits en vue d'établir si un état d'hypersensibilité a été induit.

Les observations faites suite à l'application déclenchante réalisée avec les produits utilisésà la concentration maximale non irritante, permettent de positionner en Classe 1 les sophorolipides forme brute et les sophorolipides forme acide.

Dans ces conditions, les sophorolipides forme brute et les sophorolipides forme acide présentent un pouvoir sensibilisant très faible, non significatif chez le cobaye albinos.

### 5 - Contrôles bactériologiques et étude de conservation

Un gramme de sophorolipides est dilué dans 10 ml d'eau stérile. 1ml de cette solution est alors ensemencé sur géloses Sabouraud (SAB).

Les ensemencements sont mis à l'étuve à 37°C, 120 heures ; les colonies obtenues sont dénombrées en UFC (Unités Formant Colonie) et ramenées à 1 g/ml de produit.

L'étude de conservation bactériologique du produit a été réalisé sur 12 semaines (voir tableau 3 ci-après).

Les résultats obtenus sont conformes aux normes bactériologiques.

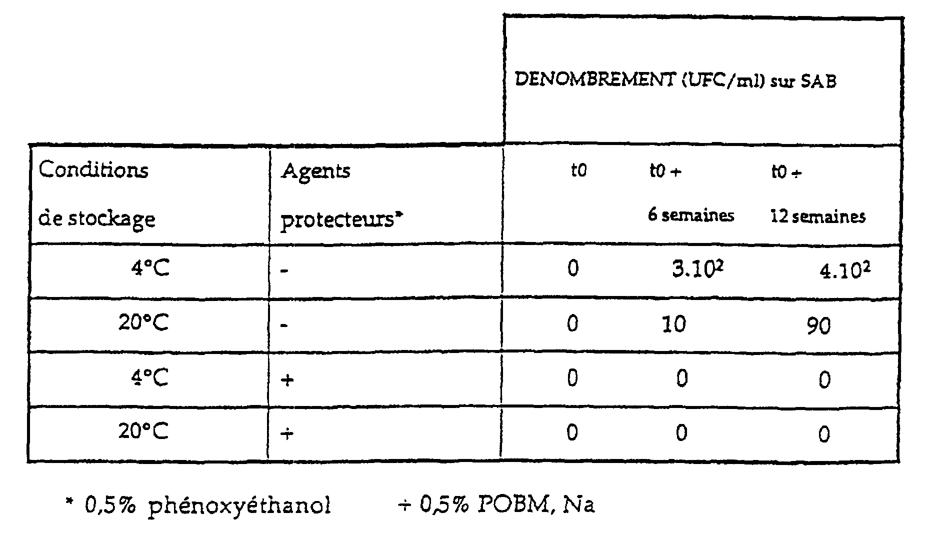

### Exemple 3 : Propriétés anti-élastasiques

L'élastase leucocytaire humaine (ELH) fait l'objet de nombreuses études, puisqu'elle intervient dans les pathologies inflammatoires, telles que l'emphysème pulmonaire, la polyarthrite rhumatoide et les parodonthopathies. Cette enzyme est capable de dégrader de nombreuses macromolécules du tissu conjonctif : l'élastine fibreuse, certains types de collagéne, les protéoglycanes et les glycoprotéines de structure. Il parait donc indispensable d'utiliser les substances anti-élastase leucocytaire, afin d'empêcher la dégradation de toutes ces macromolécules lors de phénomènes inflammatoires.

### Test d'inhibition de l'élastase leucocytaire humaine (ELH) :

Les propriétés anti-élastasiques des sophorolipides en solution aqueuse ont été étudiées en utilisant l'ELH et le N-méthoxysuccinyl-alanine-alanine-proline-valine-P-nitroanilide, comme substrat synthétique, dans un tampon TRIS HCl.
Le suivi de la cinétique a été réalisé par spectrophotométrie à 410 nm, afin d'étudier la libération de la P-nitroaniline (produit formé coloré). Les résultats sont indiqués dans le tableau 4 suivant :
Inhibition de l'élastase leucocytaire humaine par les sophorolipides forme brute

| Sophorolipides (matière sèche)mg/ml | % inhibition par rapport au témoin |
|---|---|
| 0,15 | 75 |
| 0,12 | 70 |
| 0,06 | 56 |
| 0,03 | 38 |
| 0,023 | 20 |
| 0 | 0 |

### Exemple 4 : Propriétés anti-élastasiques (sur coupe de derme humain)

- L'activité anti-élastasique des sophorolipides forme brute a été démontrée sur coupe de derme humain.
   L'élastine substrat, est celle existant dans la coupe de derme de peau humaine. L'élastase digère l'élastine (mise en évidence par histo-chimie) et l'inhibition de l'enzyme se traduit par une diminution de cette digestion.

La molécule de référence, le chlorure de mercure inhibe l'activité de l'élastase de façon dose-dépendante. Ce résultat était attendu et permet de valider l'essai.

L'évaluation de l'activité anti-élastase des sophorolipides forme brute, sur coupe de derme humain est montrée dans le tableau 5.

| Composé | Concentration % (p/v) | Dégradation des fibres élastine |
|---|---|---|
| Témoin tampon | 0 | Aucune |
| Témoin élastase | 0 | Totale |
| Sophorolipides forme brute | 0,5 | Partielle |
| | 5 | Aucune |
| Hg Cl₂ | 0,125 | Aucune |

A 5% (p/v), les sophorolipides forme brute inhibent totalement la dégradation des fibres d'élastine.

### Exemple 5 : Activité anti-élastasique (test en milieu semi-solide)

Principe : Inhibition de la dégradation de l'élastine contenue dans la gélose par l'élastase.

L'activité anti-élastase des sophorolipides forme acide, a été évaluée grâce à une technique semi-quantitative. La solution enzymatique, associée ou non au composé à l'essai ou à la molécule de référence à été déposée dans des puits creusés dans une gélose contenant le substrat : l'élastine.
L'enzyme a diffusé dans la gélose et a digéré l'élastine.
Ce phénomène se traduit par éclaircissement de la gélose dans la zone de digestion, zone qui a été mesurée.

Les effets des sophorolipides forme acide sur la digestion de l'élastine par l'élastase pancréatique sont montrés dans le tableau 6.

| Sophorolipides forme acide (%) (p/v) | Pourcentage de digestion par rapport au témoin enzyme seule (cm²) | |
|---|---|---|
| | incubation | |
| | 6 heures | 21 heures |
| 0 | 100% | 100% |
| 0,4 | 74% | 67% |
| 2 | 63% | 53% |

| Concentration de l'inhibiteur de référence (HgCl₂) | Diamètre de la zone de digestion (cm) | |
|---|---|---|
| | Incubation | |
| | 6 heures | 21 heures |
| 10 mM | 0 | 0 |

L'inhibiteur de référence testé en parallèle est le chlorure de mercure à 10mM. En présence de cette molécule, l'inhibition enzymatique est totale (tableau 7 ci-avant).

En présence du produit à l'essai seul, l'aspect de la gélose n'est pas modifiée.
En présence des sophorolipides forme acide, la surface de digestion était diminuée par rapport au témoin pour les concentrations 0,4% et 2%. Après 6 heures d'incubation, ces surfaces sont de 0,28 cm² et 0,24 cm² respectivement, pour les concentrations 0,4% et 2%. Elle est de 0,38 cm² pour le témoin sans produit. Après 21 heures d'incubation, ces surfaces sont de 0,64 cm² et 0,5 cm² respectivement pour les concentrations 0,4% et 2%. Elle est de 0,95 cm² pour le témoin enzyme sans produit.

En conclusion, dans cette étude, les sophorolipides forme acide à la concentration de 0,4% inhibent légèrement l'activité de l'élastine. Cette activité est plus fortement inhibée par le produit à la concentration de 2%. Ce produit peut donc revendiquer une activité anti-élastase à ces concentrations.

### Exemple 6 : Activité anti-radicalaire (méthode RPE)

L'effet anti-radicalaire des sophorolipides forme brute et forme acide a été évalué vis-à-vis du radical libre dérivé de l'oxygène particulièrement importants en pathologie, le radical hydroxyle.

La technique spectrométrique de la résonance paramagnétique de l'électron (RPE) a été utilisée.
Dans cette méthode les états de spin des électrons célibataires des molécules, en particulier dans les radicaux libres, sont caractérisés.

Une molécule piégeuse de radicaux libres diminue le signal.
- Les mesures RPE ont été mesurées avec un spectromètre Brucker ECS106 à température ambiante.
- Le radical hydroxyle (OH') a été produit par photolyse (U.V.B) du peroxyde d'hydrogène en solution aqueuse.
   Le mannitol est utilisé comme molécule de référence (témoin positif).

En présence de mannitol à 10mM, le signal RPE est fortement diminué : l'activité de piégeage s'élève à 98%.
Les sophorolipides forme brute aux concentrations de 0,0092% et 0,028% de matière sèche (p/v) présentent un effet de piégeage de 71% et 97% respectivement. Les sophorolipides forme brute à 2,5% de matière sèche (p/v) annulent le signal RPE.
Les sophorolipides forme acide dès la concentration de 0,0092% de matière sèche (p/v) présentent un effet de piégeage de 98%.

L'effet des sophorolipides et du mannitol sur l'intensité du signal RPE correspondant au radical hydroxyle est montré dans le tableau 8

| Composé | Concentration | % protection antiradicalaire |
|---|---|---|
| Témoin | - | 0 |
| Sophorolipides forme brute | 2,5% | 100 |
| | 0,083% | 100 |
| | 0,028% | 97 |
| | 0,0092% | 71 |
| Sophoroltpide forme acide | 2,5% | 100 |
| | 0,083% | 100 |
| | 0,028% | 98 |
| | 0,0092% | 98 |
| Mannitol | 0,182% | 98 |

Les concentrations sont exprimées en pourcentage de poids de matière sèche/volume.

Les métabolites réactifs de l'oxygène, anion superoxyde (O₂⁻), radical hydroxyle (OH⁺) péroxyde d'hydrogène (H2O2) et oxygène singulet (1O2), sont responsables de réactions toxiques pour l'organisme. Produits par l'organisme lui-même ou par des agressions extérieures, ils dégradent les sucres, les protéines, les lipides et les acides nucléiques.

Les sophorolipides forme acide et forme brute diminuent de façon dose-dépendante le signal RPE du radical hydroxyle. Cet effet est intense par rapport au mannitol (la molécule de référence connue pour son pouvoir piégeur de OH⁺).

En conclusion, les sophorolipides forme brute et forme acide présentent des effets anti-radicalaires intéressants vis-à-vis du radical hydroxyle. Ces effets se manifestent à des concentrations faibles et généralement employées pour des formulations contenant des actifs cosmétiques.

### Exemple 7 : Effet anti-radicalaire (sur fibroblastes humain)

L'effet protecteur des sophorolipides forme brute contre les radicaux libres générés par les U.V.A a été démontré sur des cultures de fibroblastes.

Les tests ont été réalisés à des concentrations en sophorolipides non toxiques pour les cellules.

La molécule de référence, l'acétate de vitamine E, à la concentration de 200 µg/ml, n'est pas toxique pour les cellules en l'absence d'U.V.A et a un effet protecteur de 31% en présence d'U.V.A.
Les sophorolipides forme brute aux concentrations de 0,125 µg/ml, 0,25 µg/ml et 0,5µg /ml entraîne une diminution de la viabilité cellulaire de 18%, 14% et 15% et ont respectivement un effet protecteur de 47%, 7% et 18%.

En absence d'agent antiradicalaire la viabilité des cellules après irradiation aux U.V.A, n'est plus que de 21%.

Le tableau 9 montre l'évaluation de l'effet antiradicalaire des sophorolipides forme brute vis-à-vis des radicaux libres générés par les U.V.A.

| Effet protecteur | Témoin exposé aux uva | Acétate de vitamine E 200µg/ml | Sophorolipides forme brute | |
|---|---|---|---|---|
| | | | 0,125µg/ml | 0,5µg/ml |
| | 0% | 31% | 47% | 18% |

Il apparaît que les sophorolipides forme brute protègent de façon efficace et remarquable les cellules vis-à-vis des radicaux libres générés par les U.V.A.

### Exemple 8 : Applications cosmétiques à base de sophorolipides

### 1. Exemples de formulations

Les tableaux 10 et 11 reprennent différents types de formulations possibles. Les teneurs sont indiquées en poids par rapport au poids total de la composition.

### 2. Mode d'introduction des sophorolipides dans des émulsions

Les sophorolipides, forme acide ou brute, peuvent être à la fois introduits dans la phase huileuse et dans la phase aqueuse.

Si l'on souhaite les introduire dans la phase huileuse, il faut les dissoudre dans la phase huileuse à 70°C, juste avant l'émulsification dans la phase aqueuse.

Dans les lotions, les shampooings ou les gels, les sophorolipides peuvent être introduits en fin de fabrication, à froid.

Dans les lotions ou les shampooings, les sophorolipides peuvent être introduits en fin de fabrication, à froid.

### 3. Exemples de préparations de formulations :

Dans les formulations ci-après, les abréviations suivantes sont utilisées:
- POBM: Parahydroxybenzoate de méthyle (conservateur)
- PCL: pure cellin
- phénonip : phénoxyéthanol, méthylparaben, éthylparaben, propylparaben, butylparaben (conservateurs).
- PPG: Propylène glycol
- Ozokérite: (cire minérale de pétrole: épaississant)
- CAO: Huile de ricin (castor oil)

### 3.1 - Gel démaquillant.

A -
   - Sophorolipides 3 % (soit 0,9% de matière active)
B -
   - Hydroxypropyl Guar
      Hydroxypropyl trimonium chloride 0,3 %
   - Hydroxyéthyl cellulose 1 %
   - Eau 84,5 %
C -
   - Sodium méthyl cocoyl taurat 0,4 %
   - Eau 10 %
D -
   - Phenonip 0,5 %
   - Nonoxynol 10 0,2 %
   - Parfum 0,10 %

Mode opératoire :
- Mélanger la phase C en chauffant modérément.
- Hydrater la phase B et y ajouter progressivement la phase C.
- Préparer la phase D, la mélanger à la préparation BC et enfin ajouter les sophorolipides.
- Mélanger jusqu'à l'obtention d'un gel homogène.

### 3.2 - Crème hydratante aux sophorolipides émulsion du type huile dans l'eau.

A -
   - Sophorolipides forme brute ou acide 2 % (soit 0,6% de matière active)
   - Phenonip 0,5 %
   - Parfum 0,2 %
B -
   - Stéarate de glycérol 4 %
   - PEG 150 distearate 0,5 %
   - Alcool cetearyl 2 %
   - Huile de germe de tournesol 2 %
   - Octanoate cetearyl 1 %
   - Triglycerides 8 %
   - Dimethicone 0,5 %
C-
   - Eau 55,70 %
D -
   - Glycérine 3 %
   - Carbomer 840 0,3 %
   - Triethanolamine 0,3 %
   - Eau 20 %

Mode opératoire :
- Préparer la phase D : ajouter dans l'ordre la glycérine, le carbomer et l'eau puis la triethanolamie.
- Faire chauffer séparément B et C (65 °C) et ajouter progressivement B dans C sous agitation.
- Ajouter D puis diminuer la température à 30-35°C, ajouter alors les sophorolipides ainsi que le conservateur et le parfum.

### 3.3. Crème hydratante aux sophorolipides du type émulsion eau/huile (HLB de 4 à 7)

Formule (calculée pour 50% d'huile végétale):
- Sel de calcium de sophorolipide obtenu à partir de:
   · Sophorolipides forme acide 10 % (4 % de matière sèche)
   · Ca(OH)₂ 10 %
- PPG** (agent stabilisant) 10 %
- Huile triglycéride de tournesol, de noix de coco hydrogénée** 50 %
- Sulfate de magnésium (parahydroxybenzoate de méthyle) 4 %
- POBM (parahydroxybenzoate) 0.6 %
- Phénoxyéthanol 0,6 %
- Kathon 0,2 %
- Parfum 0,2 %
- Eau 14,4 %

Mode opératoire:
- on chauffe l'eau à 70°C;
- on ajoute les sophorolipides et le Ca(OH)₂ puis le sulfate ;
- on fait fondre les huiles (**) puis on les incorpore dans l'eau;
- on ajoute les conservateurs POBM, Phénoxyéthanol, à 40°C et on ajoute le Kathon et le parfum.
   Des essais à 20%, 40% et 50% d'huile de noix de coco ont été réalisés.

### 3.4. Mélange de gras du type rouge à lèvres (HLB de 4 - 5)

Formule:
- Sel de calcium de sophorolipides forme brute ou forme acide. 4,0 %
- Arôme framboise 0,4 %
- Huile de ricin 39,2 %
- Merystate d'isopropyle 13 %
- PCL liquide 7,6 %
- Cire d'abeille blanche 3,4 %
- Cire de *Candelila* 7,2 %
- Cire de *Carnauba* 2,3 %
- Ozokerite 5,4 %
- Bintome CAO (gélifiant) 6,9 %
- Amerlate W (dérivé de lanoline) 7,6 %
- Ceraphyl 424 (ester gras) 3 %
- propylparaben 0,10 %

Dans cette formulation, les propriétés émulsifiantes se traduisent par le caractère d'agent mouillant du sophorolipide sous forme de sel divalent alcalin à HLB faible.

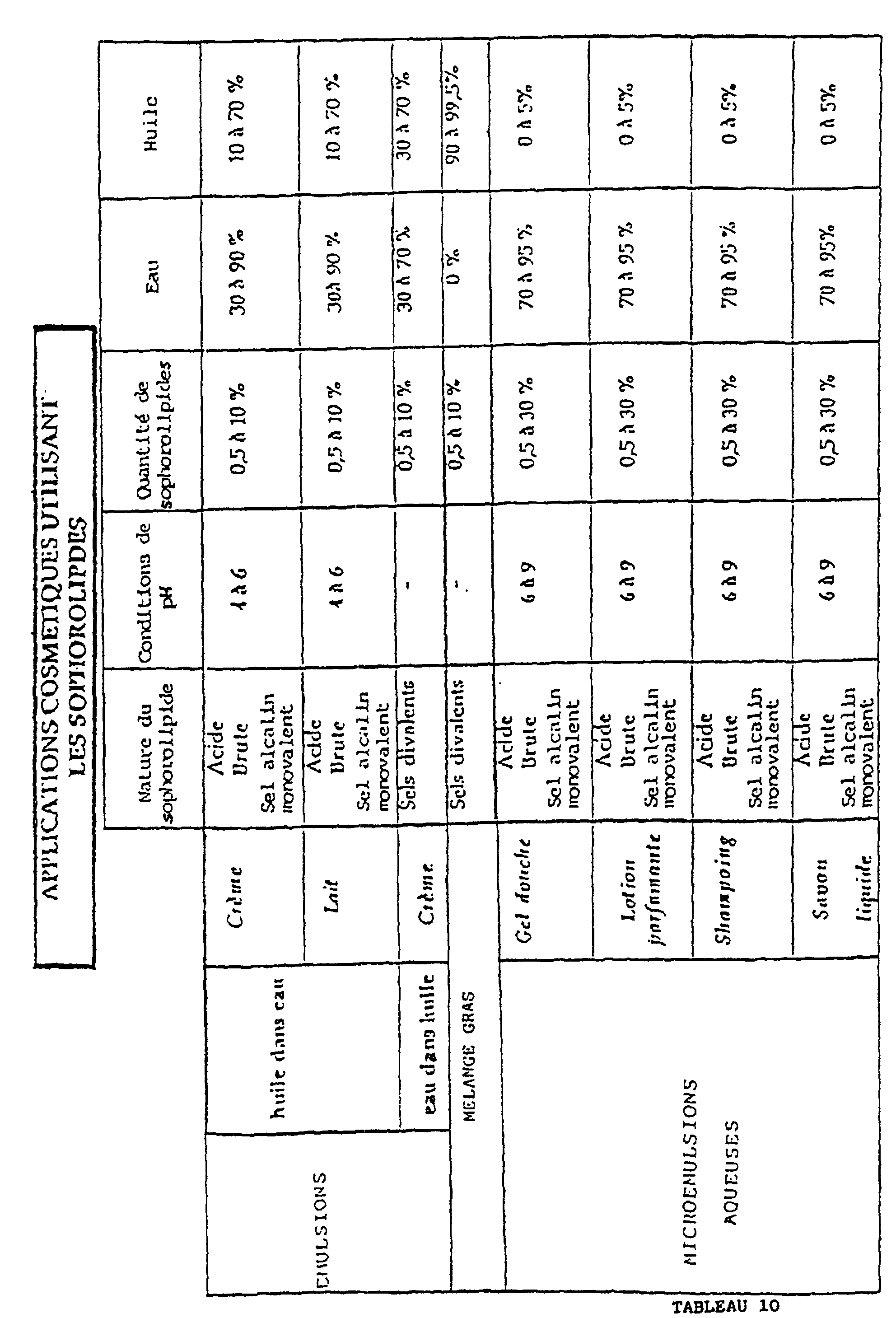

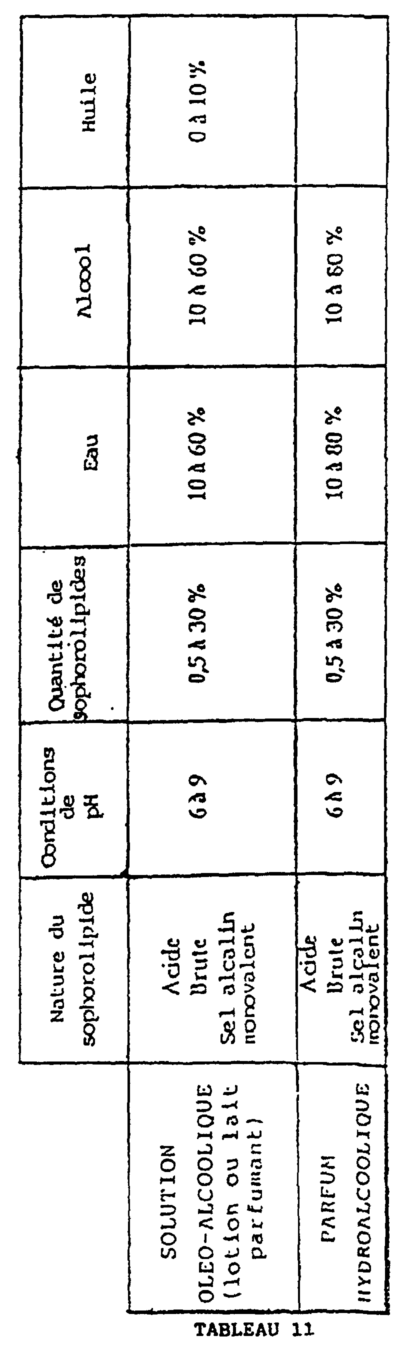

## Revendications

1. Utilisation cosmétologique d'une composition comportant au moins un sophorolipide, **caractérisée en ce que** ladite composition est utilisée en tant qu'agent anti-radicalaire, anti-élastasique et/ou anti-inflammatoire.

2. Utilisation selon la revendication 1, **caractérisée en ce que** la composition se présente sous forme d'une émulsion d'eau dans l'huile, une émulsion d'huile dans l'eau ou microémulsion d'huile dans l'eau.

3. Utilisation selon la revendication 2, **caractérisée en ce que** la composition présente sous forme d'une émulsion à phase continue huileuse dans laquelle la phase oléophile est une huile végétale telle que l'huile d'olive, l'huile d'amande douce, l'huile de noix, l'huile de coco hydrogénée.

4. Utilisation selon l'une des revendications 2 ou 3, **caractérisée en ce que** la composition comporte comme seul agent émulsifiant un sophorolipide.

5. Utilisation selon l'une des revendications 2 ou 3, **caractérisée en ce que** la composition comprend un sophorolipide et un agent émulsifiant conventionnel.

6. Utilisation selon la revendication 1, **caractérisée en ce que** le sophorolipide est dissous dans une phase huileuse, une solution aqueuse, alcoolique, oléoalcoolique ou hydroalcoolique.

7. Utilisation selon l'une des revendications 1 à 6, **caractérisée en ce que** le sophorolipide est sous forme acide protonée, sous forme acide au moins en partie sous forme de sels métalliques, sous forme brute (c'est-à-dire sous forme d'acides et/ou d'acides au moins en partie sous forme de sels métalliques, monovalents ou divalents, mélangés à des lactones).

8. Utilisation selon l'une des revendications 1 à 7, **caractérisée en ce que** la concentration en poids de sophorolipide (matière sèche) varie de 0,01 à 30 % en poids de la composition totale et de préférence 1 à 15 %.

9. Utilisation selon l'une des revendications 1 à 8 **caractérisée en ce que** la composition comporte en outre au moins un acide gras saturé ou non, à nombre de carbones compris entre 10 et 34, de préférence 14 à 22, dans une proportion pondérale de 0,01 à 20 % par rapport aux sophorolipides (matière sèche), et de préférence entre 1 et 10 %.

10. Utilisation selon l'une des revendications 1 à 9 **caractérisée en ce que** la composition consiste en une lotion, notamment parfumante, un shampooing, un gel de douche, un savon liquide, un lait, une crème, une pommade ou un mélange gras solide.

## Patentansprüche

1. Kosmetologische Verwendung einer Zusammensetzung, die wenigstens ein Sophorolipid umfasst, **dadurch gekennzeichnet, dass** die Zusammensetzung als Antiradikal-, Antielastase- und/oder Antientzündungsreagenz verwendet wird.

2. Verwendung nach Anspruch 1, **dadurch gekennzeichnet, dass** die Zusammensetzung in Form einer Emulsion von Wasser in Öl, einer Emulsion von Öl in Wasser oder Mikroemulsion von Öl in Wasser vorliegt.

3. Verwendung nach Anspruch 2, **dadurch gekennzeichnet, dass** die Zusammensetzung in Form einer Emulsion mit kontinuierlicher öliger Phase vorliegt, in der die oleophile Phase ein Pflanzenöl wie Olivenöl, süßes Mandelöl, Nussöl, hydriertes Kokosöl ist.

4. Verwendung nach einem der Ansprüche 2 oder 3, **dadurch gekennzeichnet, dass** die Zusammensetzung als einziges emulgierendes Reagenz ein Sophorolipid umfasst.

5. Verwendung nach einem der Ansprüche 2 oder 3, dadurch g**ekennzeichnet,** dass die Zusammensetzung ein Sophorolipid und ein konventionelles emulgierendes Reagenz umfasst.

6. Verwendung nach Anspruch 1, **dadurch gekennzeichnet, dass** das Sophorolipid in einer öligen Phase, einer wässrigen, alkoholischen, oleoalkoholishen oder hydroalkoholischen Lösung gelöst ist.

7. Verwendung nach einem der Ansprüche 1 bis 6, **dadurch gekennzeichnet, dass** das Sophorolipid in protonierter Säureform, in Form einer Säure, die wenigstens teilweise in Form metallischer Salze ist, in Rohform vorliegt (d.h. in Form von Säuren und/oder von Säuren, die wenigstens teilweise in Form von metallischen Salzen sind, monovalent oder divalent, vermischt mit Lactonen).

8. Verwendung nach einem der Ansprüche 1 bis 7, **dadurch gekennzeichnet, dass** die Sophorolipidgewichtskonzentration (Trockenmasse) von 0,01 bis 30 Gew.-% der Gesamtzusammensetzung und vorzugsweise 1 bis 15% variiert.

9. Verwendung nach einem der Ansprüche 1 bis 8, **dadurch gekennzeichnet, dass** die Zusammensetzung außerdem wenigstens eine gegebenenfalls gesättigte Fettsäure mit einer Kohlenstoffanzahl zwischen 10 und 34, vorzugsweise 14 bis 22 in einem Gewichtsanteil von 0,01 bis 20% im Verhältnis zu den Sophorolipiden (Trockenmasse) und vorzugsweise 1 bis 10% umfasst.

10. Verwendung nach einem der Ansprüche 1 bis 9, **dadurch gekennzeichnet, dass** die Zusammensetzung aus einer vor allem parfümierenden Lotion, einem Shampoo, einem Duschgel, einer Flüssigseife, einer Milch, einer Creme, einer Pomade oder einem festen Fettgemisch besteht.

## Claims

1. The cosmetic use of a composition comprising at least one sophorolipid, **characterized in that** said composition is used as an anti-radical, anti-elastasic and/or anti-inflammatory agent.

2. Use according to claim 1, **characterized in that** the composition is in the form of a water-in-oil emulsion, an oil-in-water emulsion or an oil-in-water microemulsion.

3. Use according to claim 2, **characterized in that** the composition is in the form of an emulsion with a continuous oily phase in which the oleophilic phase is a vegetable oil such as olive oil, sweet almond oil, walnut oil, or hydrogenated coconut oil.

4. Use according to any one of claims 2 or 3, **characterized in that** the sole emulsifying agent which the composition contains is a sophorolipid.

5. Use according to claim 2 or claim 3, **characterized in that** the composition comprises a sophorolipid and a conventional emulsifying agent.

6. Use according to claim 1, **characterized in that** the sophorolipid is dissolved in an oily phase, an aqueous solution, an alcoholic solution, an oleoalcoholic solution, or a hydroalcoholic solution.

7. Use according to any one of claims 1 to 6, **characterized in that** the sophorolipid is in a protonated acid form, in an acid form which is at least partially in the form of metallic salts, or in its crude form (i.e., in the form of acids and/or acids which are at least partially in the form of metallic salts, which are monovalent or divalent, mixed with lactones).

8. Use according to any one of claims 1 to 7, **characterized in that** the concentration of sophorolipids (dry matter) is in the range 0.01% to 30% by weight with respect to the total composition weight, preferably 1% to 15%.

9. Use according to any one of claims 1 to 8, **characterized in that** the composition further comprises at least one fatty acid which may or may not be saturated, with a number of carbons which is in the range 10 to 34, preferably 14 to 22, in a proportion of 0.01% to 20% by weight with respect to the sophorolipids (dry matter), preferably 1% to 10%.

10. Use of a cosmetic composition according to any one of claims 1 to 9, **characterized in that** the composition consists of a lotion, in particular a fragranced lotion, a shampoo, a shower gel, a liquid soap, a milk, a cream, a ointment or a solid grease mixture.
